# EUROPEAN PATENT SPECIFICATION

(11) **EP 1 814 555 B1**
(45) Date of publication and mention of the grant of the patent: **19.08.2009**
(21) Application number: 05808013.6
(22) Date of filing: 14.11.2005
(51) Int. Cl.: A61K 31/55, A61P 15/12

(54) **S-MIRTAZAPINE FOR THE TREATMENT OF HOT FLUSH**
S-MIRTAZAPIN ZUR BEHANDLUNG VON HITZEWALLUNGEN
S-MIRTAZAPINE POUR LE TRAITEMENT DES BOUFFÉES DE CHALEUR

(30) Priority: 15.11.2004 EP 04105778
(43) Date of publication of application: 08.08.2007
(73) Proprietor: N.V. Organon, 5349 AB Oss (NL)
(72) Inventor: PEETERS, B.W.M.M., Bernardus, P.O. Box 20, NL-5340 BH Oss (NL); ADANG, Anton Egbert Peter, P.O. Box 20, NL-5340 BH OSS (NL)
(74) Representative: Broekkamp, Chris L. E.
(86) International application number: PCT/EP2005/055947
(87) International publication number: WO 2006/051111

(56) References cited:
- WALDINGER, M. D. ET AL.: "Treatment of hot flushes with mirtazapine: four case reports" MATURITAS, vol. 36, 2000, pages 165-168, XP002310474
- PEREZ, D.G. ET AL.: "Pilot evaluation of mirtazapine for the treatment of hot flashes" THE JOURNAL OF SUPPORTIVE ONCOLOGY, vol. 2, no. 1, February 2004 (2004-02), pages 50-56, XP001204198
- BERENDSEN HEMMIE H G ET AL: "Oestradiol and mirtazapine restore the disturbed tail-temperature of oestrogen-deficient rats." EUROPEAN JOURNAL OF PHARMACOLOGY, vol. 482, no. 1-3, 15 December 2003 (2003-12-15), pages 329-333, XP008059771 ISSN: 0014-2999
- MALAGUENO, F.J. ET AL.: "New method for the chiral evaluation of mirtazapine in human plasma by liquid chromatography" JOURNAL OF CHROMATOGRAPHY B, vol. 809, October 2004 (2004-10), XP002310476
- SIPE K ET AL: "Serotonin 2A receptors modulate tail-skin temperature in two rodent models of estrogen deficiency-related thermoregulatory dysfunction" BRAIN RESEARCH, AMSTERDAM, NL, vol. 1028, no. 2, 3 December 2004 (2004-12-03), pages 191-202, XP004622474 ISSN: 0006-8993
- KALKMAN, H.O.: "Is migraine prohpylactic activity caused by 5-HT2B or 5-HT2C receptor blockade?" LIFE SCIENCES, vol. 54, no. 10, 1994, pages 641-644, USA
- DE BOER, TH. ET AL: "Neurochemical and autonomic pharmacological profiles of the 6-aza-analogue of mianserin, ORG3770 and its enantiomers" NEUROPHARMACOLOGY, vol. 27, no. 4, 1988, pages 399-408, Great Britain
- DE BOER, TH.: "The pharmacologic profile of mirtazapine" JOURNAL OF CLINICAL PSYCHIATRY, vol. 57, no. suppl.4, 1996, pages 19-25,
- KOOYMAN, A.R. ET AL: "Interaction between enantiomers of mianserin and ORG3770 at 5-HT3 receptors in cultured mouse neuroblastoma cells" NEUROPHARMACOLOGY, vol. 33, no. 3/4, 1994, pages 501-507, Great Britain

## Description

The invention relates to pure S-mirtazapine for use in the treatment of hot flush.

A well-known complaint during and after menopause in women is the symptom of hot flash, hot flush or night sweats. These terms refer to sudden feelings of heat or burning which starts in the head and neck area and then passes, often in waves, over the entire body. Immediately thereafter objective signs of body heat dissipation by sweating and peripheral vasodilation are usually observed (Freedman; Physiology of hot flashes; Am. J. Human Biology, Vol 13 pp 453-464,2001).

These symptoms occur in the vast majority of postmenopausal women with a prevalence estimated to be between 60 to 80% in Western societies. The mean age of onset is approximately 51 years. The complaints also occur in ovariectomized women whereby the prevalence is approximately 90%.

Hormone replacement with estrogens is an adequate treatment, but such treatment cannot be continued to higher ages (Johnson 1998, Menopause and hormone replacement therapy. Med Clin. North Am 82: 297-320)

Non-hormonal drug treatment promises to provide a safer treatment method. Experience with several drugs are reported, of which fluoxetine, paroxetine and mirtazapine are mentioned here as examples (Heath and Plouffe, 1999, EP0943329; Stearns et al.; Paroxetine controlled release in the treatment of menopausal hot flashes 2003; JAMA Vol 289; pp 2827-2834; Loprinzi et al Phase III Evaluation of Fluoxetine for treatment of Hot Flashes, J Clin Oncology Vol 20 pp 1578-1583, 2002; Waldinger MD, Berendsen HHG, Schweitzer DH. Treatment of hot flushes with mirtazapine: 4 case studies. Maturitas 2000;36:165-168; Berendsen, Maturitas Vol 36, pp 155-164, 2000; Plouffe et al., Delaware Medical Journal 69: pp 481-482, 1997, Roth and Scher, Psycho-Oncology 7, pp 129-132, 1998, and Stearns et al., Annals of Oncology 11, pp 17-22, 2000; Berendsen, Kloosterboer Oestradiol and mirtazapine restore the disturbed tail-temperature of oestrogen-deficient rats. European Journal of Pharmacology 2003; 482: 329-333; Perez et al., The Journal of supportive oncology, vol. 2, no. 1, 2004, pp 50-56).

This invention contributes to the field that advantages are obtained by selecting the S-enantiomer of mirtazapine for the treatment of hot flush. The combination of its pharmacological properties, such as α₂ receptor blockade, selective serotonin receptor blockade (in particular 5-HT_{2C} and 5-HT_{2A} receptors), duration of action by pharmacokinetic and metabolic properties, its medical effects, such as sleep quality improvement, its anti-headache effects and its anti-depressant properties, constitute a unique mixture of properties for treatment of hot flush. One would not have expected that the strongest α₂-adrenoceptor antagonist among the two enantiomers of mirtazapine is such an effective compound for this use, the more so since the α₂ antagonist yohimbine provokes temperature increases and the α₂ agonist clonidine is reported to reduce hot flushes in women (See Freedman op cit.). Neither, one would have expected that the strongest 5-HT₂ serotonin receptor antagonist among the two enantiomers of mirtazapine is such an effective compound for this use, the more so since activation rather than antagonism of central 5-HT_{2A} receptors is reported to restore temperature rises in a rat model of ovariectomy-induced thermoregulatory dysfunction (See Sipe et al., Brain Research Vol 1028, pp 191-202, 2004). Thus, the invention provides for a medicament related to mirtazapine for use in the treatment of hot flush characterized in that the medicament comprises pure S-mirtazapine as active ingredient. In another embodiment, the invention provides for the use of S-mirtazapine for the manufacture of a medicament for the treatment of hot flush.

The effect of the treatment of hot flush by the medicament according to the invention should be understood to be an effect which can be demonstrated per individual patient and/or as a group effect. An overall group effect does not exclude that the advantageous benefit of the medicine is not obtained for all persons in a group. The treatment result can be observed as diminished frequency of hot flushes or as diminished intensity of hot flushes. The selection of S-mirtazapine for use has the advantage to improve the therapeutic window or the quality of life of the treatment. When lower doses of S-mirtazapine are effective with adverse effects at the prior art dose level of racemic mirtazapine the result is a larger therapeutic window. When side effects are diminished in comparison to the effect of the symptoms to be treated the result can be seen on improvement of the subjective assessment of quality of life or on improved patient compliance.

Since the treatment of the present invention is not based on hormone replacement these treatment agents are preferably used in those circumstances were treatment with a hormone or a hormone receptor agonist bears higher risks. Therefore, an aspect of this invention is that it makes a treatment available for hot flushes in patients at risk for hormone dependent tumour growth. Such patients are the group of patients with ovariectomy in view of estrogen dependent tumour growth.
Another aspect of the invention is that it makes a treatment available for hot flushes in patients with adverse feminizing responses to estrogens. In particular, male patients functionally or pharmacologically castrated for the purpose of removing endogenous androgens can be treated for hot flushes with S-mirtazapine.
Hot flushes not only occur as complaint during menopause, but also in certain women during specific points in time of the mensual cycle, for example before and during the days of menstruation. It is an aspect of this invention that hot flushes in those circumstances can be very well non-hormonally treated with S-mirtazapine.
The terms used in this description have the meaning according to common understanding of these terms.

S-mirtazapine can be used for the purpose according to the invention as a free base or as one or more of the commonly accepted acid addition salts. Such compounds can be used in pure form or in admixture with pharmaceutical excipients.
The meaning of pure as characteristic of pure S-mirtazapine refers to the enantiomeric purity of the total active mirtazapine ingredient in the medicine according to the invention. Purity means that the therapeutic action of mirtazapine in the medicine is based on the action of the S-mirtazapine component in the medicine, without contribution from the R-enantiomer in conteracting the symptoms of hot flush. This would be the situation if more that 80% is of the total mirtazapine content of the medicine is S-mirtazapine, although higher purity is preferred, such as at least 90%, or 95%, or 99%, or 99.5% of total mirtazapine content.
The amount of S-mirtazapine, also referred to herein as the active ingredient, which is required to achieve a therapeutic effect will, of course, vary with the particular compound, the route of administration and the age and other conditions of the recipient. The amounts of mirtazapine defined in this description refer to the amount of free base of mirtazapine, unless indicated otherwise.

A suitable daily dose will be in the range of 0.5 to 140 mg, calculated on the weight content of base, per recipient per day, preferably in the range of 1 to 30, or more preferred in the range of 1 to 10 mg of the base per recipient per day. In general, parenteral administration requires lower dosages than other methods of administration which are more dependent upon absorption. However, the daily dosages are between 0.01 and 3 mg/kg body weight of the recipient. The recipient is the woman or man receiving the dose of S-mirtazapine for treatment of hot flush.
In the case of tolerance development, treatments can be further optimalized by increasing the dose up to 5 times in the course of a chronic treatment in humans. The desired dose may be presented as one, two, three or more sub-doses administered at appropriate intervals throughout the day. A treatment may be for a single day, at discretion of the patient on an "if needed" basis or for a limited determined treatment period defined by a number of days, weeks or months.
It is not excluded that the medicine according to the invention also contains another active ingredient for combination treatment, although it is excluded that the combination is with R-mirtazapine for any combination effect.
While it is possible for the active ingredient to be administered alone, it is preferable to present it as a pharmaceutical formulation. Accordingly, the present invention further provides a pharmaceutical formulation for use in the treatment of hot flushes comprising pure S-mirtazapine, together with a pharmaceutically acceptable carrier thereof and optionally other therapeutic agents. The carrier must be "acceptable" in the sense of being compatible with the other ingredients of the formulation and not deleterious to the recipients thereof. Suitable excipients are made available *e.g.,* in the Handbook of Pharmaceutical Excipients, 2nd Edition; Editors A. Wade and P.J.Weller, American Pharmaceutical Association, Washington, The Pharmaceutical Press, London, 1994. The invention further includes a pharmaceutical formulation, as hereinbefore described, in combination with packaging material suitable for the pharmaceutical formulation, said packaging material including instructions for the use of the pharmaceutical formulation in the treatment of hot flush.

Formulations include those suitable for oral or vaginal administration. The formulations may be prepared by any methods well known in the art of pharmacy, for example, using methods such as those described in Gennaro et al., Remmington: The Science and Practice of Pharmacy, 20th Edition, Lippincott, Williams and Wilkins, 2000; see especially part 5: pharmaceutical manufacturing. Such methods include the step of bringing into association the active ingredient with the carrier which constitutes one or more accessory ingredients. Such accessory ingredients include those conventional in the art, such as, fillers, binders, diluents, disintegrants, lubricants, colorants, flavoring agents and wetting agents.

Formulations suitable for oral administration may be presented as discrete units such as tablets or capsules each containing a predetermined amount of active ingredient; as a powder or granulates; as a solution or suspension. The active ingredient may also be presented as a bolus or paste, or may be contained within liposomes or microparticles.

Formulations, which are parenteral (for example subcutaneous) may also be presented in a suitable sustained release form, for example, in a device such as the Minipump™.

S-mirtazapine can be prepared in several manners, e.g. by purification from the racemic mixture mirtazapine. Mirtazapine may be prepared using the method described in US 4,062,848. S-mirtazapine can also be obtained by stereoselective synthesis.

The following examples are for illustration

### Example 1

The effect of the maleate salt of S-mirtazapine was measured in an animal model for hot flushes. The model is based on the observation that the drop in tail skin temperature at the transition from the rest to the active phase in rats is attenuated after ovariectomy. The locomotor activity of the rats was also measured.
In rats it was found that the fall in tail skin temperature during the transition from the passive (light) to the active period (dark) was attenuated after estrogen withdrawal through ovariectomy. The difference between ovariectomized rats and sham operated rats in the active period was restored by estradiol treatment indicating that this effect on tail skin temperature is caused by estrogen withdrawal after ovariectomy.

### Materials and methods

The studies were performed in accordance with the guidelines for animal research of the Dutch government.

Tail skin temperature of the rats was measured by use of the telemetry system from Data Sciences international (DSI), a division of Transoma Medical, Inc., USA.
This system comprises implantable wireless transmitters (TA10TA-F40 W/TP), receivers (RPC-1 and RLA1020), a data exchange matrix (DEM), and a data acquisition and analysis system (DQ ART 2.2 silver version).
The transmitter contains a hermetically sealed plastic housing covered with a biocompatible silastic coating, has a volume less than 3.5 cc and weighs approximately 7 g.
Each transmitter contains an amplifier, a battery, radio-frequency electronics (for telemetric transmission of temperature and activity data), and a magnetically activated switch which allows the device to be turned on and off. The transmitter contains a 13 cm long thermistor probe with a modified temperature tip.

The S-mirtazapine maleate was dissolved in 0.9% saline with 5% mulgofen™. Compound and vehicle were administered intraperitoneally. Sham placebo and ovariectomized(OVX)/placebo groups received 2 ml/kg/day saline and compound treated groups received 10, 30 or 100 mg/kg/day in a volume of 2 ml/kg/day. Here, the indicated amounts refer to the weight of the maleate salt of S-mirtazepine.

Female rats from the Crl:WU strain, weighing 225-250 g at arrival, were supplied by CPB Harlan, The Netherlands. Rats were housed individually in Macrolon^{™} cages (38 x 22 x 15 cm) with a sawdust bedding and "Enviro Dry^{™}" as cage enrichment. The day after arrival the rats were ovariectomized or sham operated and the transmitter was implanted intraperitoneally under isoflurane aneasthesia. After surgery the rats were placed in a temperature controlled room (21.5 ± 1 °C) with an adapted light/dark cycle (lights off 9:30h a.m., lights on 7:30h p.m.). They were allowed to recover from surgery and to adapt to the light/dark cycle for at least 2 weeks. Food and water were given ad libitum.
Rats were re-used in the consecutive experiments with an interval of at least 2 weeks between the treatment periods.

The transmitters TA10TA-F40 W\TP were sterilized by placing them in 2% glutaraldehyde for about 16 hours. They were rinsed and kept in 70% ethanol until implantation. Prior to implantation they were rinsed with sterile saline.

The left and right flank and the rear end of the back above the tail implant of the rat were shaved. The rats were anaesthetized with isoflurane (FORENE^{®}/1-chloro-2,2,2,-trifluorethyl-difluoromethyl-ether, Abbott). The operation area was disinfected with Hibicet^{®} (1.5% m/v chloorhexidinedigluconaat and 1.5% m/v cetrimide). The rats were bilateral ovariectomized (OVX) or sham operated. The incision made in the right flank was also used to implant the transmitter in the peritoneal cavity. When closing the abdomen the transmitter was fixed to the abdominal muscles with stitches.
On the back of the rat in height of the hips, a small incision in the skin was made and the thermistor-tipped probe was led to the back of the rat. From here the probe was brought under the skin in the tail to about 2 cm from the base of the tail. The lead was fixed with a stitch at the musculus biceps femoris. To complete the operation the skin was closed with wound clips. Immediately after surgery the rats were treated with the analgesic buprenorphine 50 µg/kg sc (Temgesic^{®}, Schering-Plough).

Treatment groups were randomized over the OVX groups. The rats were injected intraperitoneally with vehicle or S-mirtazapine maleate once a day at 11:00 a.m. for a period of 5 days. The tail skin temperature and the locomotor activity of the rats were measured every 5 minutes continuously, but the measurements between 12:00 and 02:00 pm (in which period the maximal effect of S-mirtazapine was observed) were used for calculations of the treatment effects. During this period the rats remained completely undisturbed. Before 9:30 am (the time the light turned off) the rats could be taken care of.
Body weights of the rats were determined before and after the treatment period.
Data were expressed as mean ± S.E.M. unless otherwise specified. For testing statistical significance the Analysis of Variance (ANOVA) was used. The data were logarithmically transformed to normalize variations. A value of P<0.05 was considered to be significant.
Data were collected for each rat for 7 seconds every 5 minutes during two hours starting one hour after drug administration (11:00 a.m.). The mean tail skin temperature over this 2-h period was calculated for each rat. In addition, the group means were calculated together with the standard error of the mean (SEM). The tail skin temperature is expressed in degrees centigrade (°C) and locomotor activity in counts per minutes (cpm).
The number of animals in the different test groups varied between 5-8.

### Results

The results show the effects of treatments on the tail skin temperature (TST) and the locomotor activity of ovariectomized rats.
The results show that 30 mg/kg, but not 10 mg/kg S-mirtazapine maleate restored the attenuation of the OVX-induced drop in tail skin temperature. Locomotor activity was reduced after 30 mg/kg, but this effect was not statistically significant.

In experiment 1 S-mirtazapine maleate was tested in a dose of 10 mg/kg/day intraperitoneally for 5 days.
Data were obtained on the mean ± SEM of the tail skin temperatures of OVX rats at start (the mean of the three days before starting treatment), during administration of S-mirtazapine maleate at 10 mg/kg/day i.p. and during two days after stopping treatment. S-mirtazapine maleate showed an increase in TST of approximately 2 °C after the first treatment day and stayed at a significantly higher level during the treatment period as compared to sham/placebo and compared to OVX/placebo at day 4. On day 5 S-mirtazapine maleate had no effect on the tail skin temperature. After the end of treatment TST of all groups returned to the normal values for OVX rats of about 29 °C. The locomotor activity of the sham operated and the ovariectomized rats treated with vehicle fluctuated around 1.0 counts per minute (cpm) during the experiment.
S-mirtazapine maleate showed a significant reduction on locomotor activity when compared to the sham operated placebo group on day 3.
When compared to the OVX placebo group S-mirtazapine maleate showed no significant effect on locomotor activity at 10 mg/kg i.p.. and had no significant effect on the body weight (BW) of the treated rats during the experiment.

In experiment 2 S-mirtazapine maleate was tested in a dose range of 10, 30 and 100 mg/kg/day intraperitoneal for 5 days.
At 10 mg/kg) caused an increase in TST of 1 - 3 °C after the first treatment and stayed at a significantly higher level at day 2 and 3 during the treatment period when compared to the OVX rats. After end of treatment TST returned to normal TST values of OVX rats of about 28-29 °C.
At 30 and 100 mg/kg i.p. significantly restored the fall in TST. From day 2 onwards S-mirtazapine maleate at 30 and 100 mg/kg i.p. showed a lower TST (2-4 °C) than the sham operated group. At 30 mg/kg i.p. on day 5 the TST was statistically significantly different from the sham operated placebo group.

After the end of treatment the TST of the 30 mg/kg i.p. group returned to normal values of OVX rats while the TST of the 100 mg/kg i.p. group stayed up to 2 days after the end of treatment at a lower level.
After treatment for 5 consecutive days the lowest dose (10 mg/kg) showed no effect compared to the OVX rats. At 30 and 100 mg/kg i.p. a significant decrease of the tail skin temperature compared to the OVX group. The TST of the 30 mg/kg i.p. group was also statistically significantly below the sham operated group.

The locomotor activity of the sham operated and the OVX rats treated with vehicle fluctuated around 1.2 cpm during the experiment. Treatment of OVX rats with 10 mg/kg/day S-mirtazapine maleate showed only an effect on locomotor activity after the first treatment. During the rest of the experiment the locomotor activity of the rats remained at the level of the placebo treated OVX rats.
S-mirtazapine maleate administered at doses of 30 and 100 mg/kg i.p. caused a decrease in activity to about 0.5 cpm. The decrease in locomotor activity was seen from the first treatment onwards. At the end of the treatment period the locomotor activity of the rats treated with 30 mg/kg i.p. S-mirtazapine maleate remained at the level around 0.5 cpm while the locomotor activity of the rats treated with 100 mg/kg i.p. increased to the level of the vehicle treated rats. No statistically significant effect of S-mirtazapine maleate after 5 days treatment was seen in any dose group when compared to the OVX placebo group although at 30 mg/kg i.p. a 50% drop in locomotor activity was observed.
Conclusion: This method demonstrates an effect against hot flush at dose levels of 30 and 100 mg/kg in rats. The model can be extrapolated to human recipients, although the exact dose level is not predictive for the suitable dose level in humans. In general rats tend to show the relevant effects at much higher dose levels than the dose levels needed in humans.

### Example 2

### Human recipients

The beneficial effects of treatment with S-mirtazapine maleate can be demonstrated with the following method.
Patients are postmenopausal women, defined as either: 12 months of spontaneous amenorrhea or 6 months of spontaneous amenorrhea with serum FSH levels > 40 mIU/mL or 6 weeks post-surgical bilateral oophorectomy with or without hysterectomy.

In case the menopausal status of a subject is unclear because of a hysterectomy, the serum FSH level must be > 40 mIU/mL. If the date of the last menstruation is not dear because of perimenopausal hormone use, then the subject must have a serum FSH level > 40 mIU/mL after completion of a washout period. Only recipients within the age group of 40 - 65 years and with body mass index between 18 and 32 are included in the test. Patients have at least 7 moderate to severe hot flushes per day or 50 per week, as quantified from daily diary recordings during at least 7 days preceding randomization to trial medication.
Groups of patients receive dosage units containing 2.25, 4.5, 9 or 18 mg of S-mirtazapine (weight of the base) in the form of S-mirtazapine maleate as encapsulated tablets with maize starch and lactose monohydrate as major excipients for oral intake. Subjects are instructed to take one capsule in the evening prior to sleep.

### Definitions:

Baseline: the last assessment before first drug administration
In-treatment period: the period from first up to and including last drug administration plus one day.
The frequency and severity of hot flushes is evaluated via electronic diaries completed daily by the subjects during the pretreatment and treatment periods. The severity of hot flushes is rated by subjects as mild, moderate, severe using the following reference definitions:
Mild : sensation of heat without sweating
Moderate : sensation of heat with sweating, able to continue activity
Severe: sensation of heat with sweating, causing cessation of activity
If no hot flushes were experienced, this is to be recorded as 'no sensation of heat.

Efficacy can be determined by recording vasomotor complaints recorded by the subject using an electronic diary card (pad). The number and severity (mild, moderate, or severe) of vasomotor symptoms need to be recorded by the subjects on a daily basis during screening and during the full treatment period of 12 weeks.

At the baseline visit the frequency of moderate to severe hot flushes will be examined for the last seven complete days preceding the baseline visit in order to confirm the subject's eligibility for inclusion into the test. Baseline scores are based on the last 7 days of non-missing vasomotor symptoms recorded before randomization.

For each week, the total number of moderate to severe vasomotor symptoms, a Severity Score A, and a Composite Score A can be calculated using the formulae as presented below:
**Frequency score A** = (number of moderate vasomotor symptoms) + (number of severe vasomotor symptoms)
**Severity score A** = (number moderate vasomotor symptoms) x 1 + (number severe vasomotor symptoms) x 2 divided by the Total number of moderate/severe vasomotor symptoms
**Composite score A** = (number moderate vasomotor symptoms) x 1 + (Number severe vasomotor symptoms) x 2
   Note that the Composite score A equals Severity score A times Frequency score A and hence the composite score A is particularly large for subjects experiencing many severe vasomotor symptoms.

The following efficacy variables based on mild to severe vasomotor symptoms can also be derived and analyzed:
**Frequency score B** = (Number of mild vasomotor symptoms) + (Number of moderate vasomotor symptoms) + (Number of severe vasomotor symptoms)
**Severity score B** = (Number of mild vasomotor symptoms) + (Number moderate vasomotor symptoms) x 2 + (Number severe vasomotor symptoms) x 3 divided by the Total number of vasomotor symptoms
**Composite score B** = (Number of mild vasomotor symptoms) + (Number of moderate vasomotor symptoms) x 2 + (Number of severe vasomotor symptoms)x3
   The frequency of vasomotor symptoms, the severity scores A and B, and the composite scores A and B will not be calculated for days with missing vasomotor symptoms data for all levels of severity relevant for that score (otherwise 0 will be assumed). When the total number of relevant vasomotor symptoms equals zero, the associated composite severity score will be set to missing.
   Please note that the Composite score B equals the Severity score B times Frequency score B.
   Both the frequency scores A and B, and the composite scores A and B will be expressed as daily averages (by dividing by the number of days with non-missing information). The severity scores A and B, on the other hand, are dimensionless and will not need to be divided as such. Moreover, these are purposely not calculated on a daily basis (and then averaged) since such daily severity scores would be highly sensitive to low daily frequencies and hence would largely influence the average.

### Primary efficacy variables

The 4 co-primary efficacy parameters can be, in order of hierarchy, the changes from baseline of the
1. Average daily frequency of moderate and/or severe hot flushes at Week 4,
2. Average daily frequency of moderate and/or severe hot flushes at Week 12 (maintenance),
3. Severity score B at Week 4, and
4. Severity score B at Week 12;
   during the available in-treatment days at which vasomotor symptoms were recorded of the respective week using the last-observation carried forward (LOCF) approach.

The required baseline values are determined as, respectively, the average daily number of moderate to severe vasomotor symptoms and the severity score B during at most 7 non-missing pre-treatment days (i.e. prior to Day 1) at which vasomotor symptoms were recorded.

### Missing data

For each Week the last 7 days with non-missing data will be used, taking data up to and including 21 days before the start of the Week (or up to Day 1, whichever limit comes first). Only if during the respective treatment week no vasomotor symptoms were recorded will the efficacy variable of the previous in-treatment week will be carried forward. Baseline values will therefore not be carried forward, but sensitivity analysis will be performed carrying forward baseline values for subjects with no post-baseline measurements.

### Secondary efficacy variable(s)

Secondary efficacy variables are change from baseline with respect to:
1) The average total daily frequency of moderate/severe vasomotor symptoms per consecutive treatment week (other than Week 4 and 12);
2) The severity score A of vasomotor symptoms per consecutive treatment week;
3) The average composite score A of vasomotor symptoms per consecutive treatment week;
4) The average total daily frequency of mild/moderate/severe vasomotor symptoms per consecutive treatment week;
5) The severity score B of vasomotor symptoms per consecutive treatment week (other than Week 4 and Week12);
6) The average composite score B of vasomotor symptoms per consecutive treatment week;
7) the number of responders, defined as subjects with a reduction of at least 50% on average daily frequency of moderate/severe vasomotor symptoms compared to baseline, per consecutive treatment week.
8) the number of remitters, defined as subjects with at most one moderate or severe hot flush per day on average, per consecutive treatment week;

### Assessment windows that can be used in the efficacy analysis

| **Assessment Week** | **Day** |
|---|---|
| Baseline | Last 7 non-missing days before Day 1 |
| Week 1 | 2^{a}-8 |
| Week 2 | 9-15 |
| Week 3 | 16-22 |
| Week 4 (Primary time point) | 23-29 |
| Week 5 | 30-36 |
| Week 6 | 37-43 |
| Week 7 | 44-50 |
| Week 8 | 51-57 |
| Week 9 | 58-64 |
| Week 10 | 65-71 |
| Week 11 | 72-78 |
| Week 12 | 79-85 |

| | |
|---|---|
| *^{a}*First capsule intake takes place in the evening of Day 1 and vasomotor symptoms recorded then are hence to be regarded as pre-treatment | |

### Other variables

### Women's Health Questionnaire

Health related quality of life can be assessed at baseline, Week 2, Week 4, Week 8 and Week 12 visits by a self-administered questionnaire (WHQ).
The WHQ consists of 36 items measured on a 4-point scale and divided into 9 subscores. Each item can be scored as follows: 'Yes definitely=1', 'Yes sometimes=2', 'No not much=3' and 'No not at all=4'. Each score is transformed to a value `1' for scores '1' and '2', and to a value '0' for scores '3' and '4'. Items 7, 10, 21, 25, 31 and 32 are phrased positively whereas the rest of the items are phrased negatively. Therefore these six items are transformed in reverse order.
The nine subscores are calculated on transformed scores as follows:
a) somatic symptoms: (sum of items 14, 15, 16, 18, 23, 30, 35) / 7
b) depressed mood: (sum of items 3, 5, 7*, 8, 10*, 12, 25*) / 7
c) memory/concentration: (sum of items 20, 33, 36) / 3
d) anxiety/fears: (sum of items 2, 4, 6, 9) / 4
e) sexual behavior: (sum of items 24, 31*^{†}, 34^{†}) / 3
f) vasomotor symptoms: (sum of items 19, 27) / 2
g) sleep problems: (sum of items 1, 11, 29) / 3
h) menstrual problems: (sum of items 17, 22, 26, 28) / 4
i) attractiveness: (sum of items 21*, 32*) / 2
   *) Items that are scored in reverse order.
   ^{†}) When both variables 31 and 34 are not entered (e.g. subject is not sexually active) the sexual behavior subscale will not be calculated.
   Each subscore therefore ranges from 0 to 1, where lower scores are better.

The nine subscores (somatic symptoms, depressed mood, memory/concentration, anxiety/fears, sexual behavior, vasomotor symptoms, sleep problems, menstrual, and attractiveness) can demonstrate different effects in treatment groups.

## Claims

1. Pure S-mirtazapine for use in the treatment of hot flush.

2. The use of S-mirtazapine for the manufacture of a medicament for use in the treatment of hot flush.

## Patentansprüche

1. Reines S-Mirtazapin für eine Verwendung bei der Behandlung von Hitzewallungen.

2. Verwendung von S-Mirtazapin für die Herstellung eines Medikamentes für eine Verwendung bei der Behandlung von Hitzewallungen.

## Revendications

1. S-mirtazapine pure pour son utilisation dans le traitement des bouffées de chaleur.

2. Utilisation de S-mirtazapine pour la fabrication d'un médicament pour son utilisation dans le traitement des bouffées de chaleur.
